(19)

Europäisches
Patentamt
European
Patent Office
Office européen
des brevets

(11)     **EP 2 336 355 B1**

(12)             **EUROPEAN PATENT SPECIFICATION**

(45) Date of publication and mention
of the grant of the patent:
**22.01.2014  Bulletin 2014/04**

(51) Int Cl.:
***C12Q 1/68*** *(2006.01)*

(21) Application number: **09820656.8**

(22) Date of filing: **13.10.2009**

(86) International application number:
**PCT/JP2009/067966**

(87) International publication number:
**WO 2010/044481 (22.04.2010 Gazette 2010/16)**

(54) **BIOLOGICAL RHYTHM PREDICTION METHOD**

VERFAHREN ZUR VORHERSAGE EINES BIOLOGISCHEN RHYTHMUS

PROCÉDÉ DE PRÉDICTION DE RYTHME BIOLOGIQUE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **16.10.2008  JP 2008267464**

(43) Date of publication of application:
**22.06.2011  Bulletin 2011/25**

(73) Proprietor: **Sony Corporation
Tokyo 108-0075 (JP)**

(72) Inventors:
• **SOMA, Haruhiko
Tokyo 108-0075 (JP)**
• **YAMAMOTO, Takuro
Tokyo 108-0075 (JP)**
• **AKASHI, Makoto
Saga-city
Saga 840-8502 (JP)**
• **NODE, Koichi
Saga-city
Saga 840-8502 (JP)**
• **KISHII, Noriyuki
Tokyo 108-0075 (JP)**
• **NAKAGAWA, Kazuhiro
Tokyo 108-0075 (JP)**
• **YAMASHITA, Shiko
Tokyo 108-0075 (JP)**
• **HAYAKAWA, Tomohiro
Tokyo 108-0075 (JP)**

(74) Representative: **Müller - Hoffmann & Partner
Patentanwälte
St.-Martin-Strasse 58
81541 München (DE)**

(56) References cited:
**EP-A1- 1 785 493        EP-A1- 2 180 047
WO-A1-2004/012128     WO-A1-2008/105476
WO-A1-2009/008374     WO-A1-2009/014036
WO-A2-2006/135733**

• **BJARNASON GEORG A ET AL: "Circadian
expression of clock genes in human oral mucosa
and skin: Association with specific cell-cycle
phases", AMERICAN JOURNAL OF
PATHOLOGY, vol. 158, no. 5, May 2001 (2001-05),
pages 1793-1801, XP002674505, ISSN: 0002-9440**
• **TAKIMOTO MIEKO ET AL: "Daily expression of
clock genes in whole blood cells in healthy
subjects and a patient with circadian rhythm
sleep disorder", AMERICAN JOURNAL OF
PHYSIOLOGY: REGULATORY, INTEGRATIVE
AND COMPARATIVE PHYSIOLOGY, AMERICAN
PHYSIOLOGICAL SOCIETY, US, vol. 289, no. 5, 1
November 2005 (2005-11-01), pages R1273-
R1279, XP002603039, ISSN: 0363-6119, DOI:
10.1152/AJPREGU.00126.2005 [retrieved on
2005-06-16]**
• **S. A. BROWN ET AL: "Molecular insights into
human daily behavior", PROCEEDINGS OF THE
NATIONAL ACADEMY OF SCIENCES, vol. 105,
no. 5, 5 February 2008 (2008-02-05), pages
1602-1607, XP55025320, ISSN: 0027-8424, DOI:
10.1073/pnas.0707772105**
• **YAMAMOTO TAKURO ET AL: "Transcriptional
oscillation of canonical clock genes in mouse
peripheral tissues", BMC MOLECULAR
BIOLOGY, BIOMED CENTRAL LTD, GB, vol. 5, no.
1, 9 October 2004 (2004-10-09) , page 18,
XP021002729, ISSN: 1471-2199, DOI:
10.1186/1471-2199-5-18**

EP 2 336 355 B1

- AKASHI MAKOTO ET AL: "Noninvasive method for assessing the human circadian clock using hair follicle cells", PROCEEDINGS OF THE NATIONAL ACADEMY OF SCIENCES, NATIONAL ACADEMY OF SCIENCES, vol. 107, no. 35, 31 August 2010 (2010-08-31), pages 15643-15648, XP002603040, ISSN: 0027-8424, DOI: 10.1073/PNAS.1003878107 [retrieved on 2010-08-23]
- CERMAKIAN N ET AL: "A MOLECULAR PERSPECTIVE OF HUMAN CIRCADIAN RHYTHM DISORDERS", BRAIN RESEARCH REVIEWS, ELSEVIER, NL, vol. 42, no. 3, 1 January 2003 (2003-01-01), pages 204-220, XP001187590, ISSN: 0165-0173, DOI: 10.1016/S0165-0173(03)00171-1
- HAYES, K.R. ET AL.: 'Circadian clocks are seeing the systemsbiology light.' GENOME BIOLOGY vol. 6, no. 5, 2005, pages 219.1 - 219.3, XP008145572
- CLARIDGE-CHANG, A. ET AL.: 'Circadian regulation of gene expression systems in the Drosophila head.' NEURON vol. 32, no. 4, 20 November 2001, pages 657 - 671, XP002974820
- TORU TAKUMI: 'Tokei Idenshi no Tensha Shindo Oyobi sono Bunshi Mechanism' THE AUTONOMIC NERVOUS SYSTEM vol. 44, no. 4, 15 August 2007, pages 246 - 250, XP008146139
- SHIGENOBU SHIBATA: 'Jikoku-Jikan ni Okeru Tainai Tokei Kiko no Yakuwari' THE JAPANESE JOURNAL OF ANIMAL PSYCHOLOGY vol. 58, no. 1, 25 June 2008, pages 21 - 31, XP008145579
- HARUHIKO SOMA ET AL.: 'Tei Hindo Kentai Saishu ni yoru Hito Tokei Idenshi Hatsugen Rhythm Iso Yosoku' JOURNAL OF CHRONOBIOLOGY vol. 14, no. 2, 20 October 2008, page 64, XP008146132

**Description**

Technical Field

The present invention relates to a method for predicting a biological rhythm of a subject. More specifically, the present invention relates to a method for predicting a biological rhythm by collecting biological specimens from a subject only three times within 24 hours.

Background Art

It is known that various biological phenomena of living individuals show a "periodical rhythm" that autonomously oscillates. This periodical rhythm is called a "biological rhythm". In particular, it is known that a "circadian rhythm" whose period is about one day widely controls biological phenomena such as a sleep/wake cycle, body temperature, blood pressure, and a diurnal variation in hormone secretion. Furthermore, a circadian rhythm is involved in the activity of mind and body, athletic ability, and the sensitivity to drugs.

A biological rhythm is controlled by a gene cluster called "clock genes". Clock genes function as an "internal clock" by autonomously causing the expression, activity, localization, or the like of the clock genes to periodically vary (oscillate).

It is obvious that the gene polymorphism and gene mutation of clock genes are the cause of the onset of cancer, diabetes, vascular diseases, neurodegenerative diseases, and the like. Furthermore, it has been pointed out recently that the gene polymorphism and mutation of clock genes are involved in the onset of mental diseases such as bipolar disorder and melancholia.

On the other hand, a biological rhythm is not only autonomously controlled by an internal clock, but also restricted by life in society. For example, in the sleep/wake cycle, there may be a difference in rhythm (phase difference) between "retiring/rising cycle in actual life" and "sleep/wake cycle caused by an internal clock" due to the change in the regular bedtime and hour of rising.

It is believed that the difference in biological rhythm causes so-called "jet lag" and a sleep disorder and furthermore the above-described mental diseases. To cure such diseases, an attempt to reset an abnormal internal clock through irradiation with light has started.

In addition, an attempt to maximize the effect of medication using a biological rhythm has started. It is believed that the effect of medication also diurnally varies due to the expression level of a molecule (drug-targeting molecule) targeted by a drug and the circadian rhythm of the activity of an enzyme (drug-metabolizing enzyme) that metabolizes a drug. Thus, there has been proposed a concept of "time medical care" that attempts to maximize a curative effect by setting administration time appropriate for each drug.

Moreover, in more familiar cases, by utilizing the circadian rhythm of the activity of mind and body and athletic ability, the activity time that maximizes one's ability in learning and training and the ingestion time that does not easily cause a gain in weight (or easily causes a gain in weight) have started to be examined.

In view of the foregoing, it is believed that precise evaluation of a biological rhythm is significantly beneficial for improving poor physical condition such as jet lag, preventing various diseases, achieving time medical care, exhibiting one's ability, and losing weight.

PTL 1 discloses a method for estimating an internal body time on the basis of the measurement data of gene expression product level of a standard specimen collected from a living individual. In this method for estimating an internal body time, a molecular clock table for estimating an internal body time is prepared on the basis of the expression level of gene expression product level (i.e. mRNA).

Prior art document WO 2009/014036 discloses a low-invasive method for obtaining information on the biological rhythm in an individual organism based on the change in expression level of a CLOCK gene in hair follicle cells in the organism over time. Based on a molecular CLOCK table and on the expression level which are checked two or more times a phase shift in the biological rhythm can be detected.

**Citation List**

PTL 1: International Publication No. 2004/012128

**Summary of Invention**

PTL 1 does not disclose a specific target gene for measurement. Biological rhythms have been conventionally estimated using a clock gene as a measurement target on the basis of a change in the expression level of the clock gene over time.

However, for the measurement of the change in the expression level of a clock gene over time, a biological specimen needed to be continuously collected from a subject. That is, for the estimation of a biological rhythm with high precision, a biological specimen needed to be collected from a subject for 24 hours at intervals of every several hours to obtain time series data of clock gene expression levels.

It is a heavy burden on a subject (subject individual) to collect a biological specimen several times a day in such a manner. Furthermore, a subject was sometimes caused to wake up at midnight to collect a biological specimen. This affected the sleep/wake cycle of the subject, which might change the biological rhythm itself.

It is believed to be important to reduce the burden on a subject imposed when a biological specimen is collected, in order to widely spread the valuation of a biological rhythm for the purpose of, for example, improving poor physical condition such as jet lag and preventing various diseases.

Accordingly, a main object of the present invention is to provide a method that achieves the prediction of a biological rhythm with high precision while minimizing the number of times a biological specimen is collected from a subject.

The object underlying the present invention is achieved by means of a method for predicting a biological rhythm of a subject as defined in independent claim 1. Preferred embodiments of the method are defined in the dependent claims.

To achieve the above-described object, the present invention provides a method for predicting a biological rhythm of a subject on the basis of time series expression level data obtained by measuring expression levels of two clock genes in biological specimens collected from the subject three times within 24 hours, the clock genes having different phases of circadian cycles of a change in expression levels.

More specifically, the method for predicting a biological rhythm includes:

(1) a step of collecting a biological specimen from a subject three times within 24 hours;
(2) a step of measuring expression levels of two clock genes in the biological specimen, the two clock genes having different phases of circadian cycles of a change in expression levels; and
(3) a step of calculating the circadian cycles from time series expression level data obtained through the steps (1) and (2).

By measuring the two clock genes having different phases of circadian cycles of a change in expression levels, the number of times the biological specimen is collected from the subject can be set to be only three within 24 hours.

In the step (3) of this method for predicting a biological rhythm, the circadian cycles are calculated from the time series expression level data using formulas (I) and (II) below.

$$E_a(t) = A_a \cos\left\{\frac{2\pi(t + \varpi)}{24}\right\} + C_a$$

Formula (I)

$$E_b(t) = A_b \cos\left\{\frac{2\pi(t + \varpi + \theta)}{24}\right\} + C_b$$

Formula (II)

(In the formula (I), $E_a(t)$, $A_a$, $\omega$, and $C_a$ are an expression level, an amplitude, an initial phase, and an offset value of one of the clock genes at a time t. Moreover, in the formula (II), $E_b(t)$, $A_b$, and $C_b$ are an expression level, an amplitude, and an offset value of the other of the clock genes at a time t. Furthermore, $\theta$ is a phase difference between the two clock genes.)

This method for predicting a biological rhythm preferably includes calculating model data from a cosine curve obtained by performing cosine fitting on time series expression level data, the model data showing a change in expression level every hour; calculating three-point sampling data from three arbitrary points of time and expression levels at those points of time extracted from the model data, and the formulas (I) and (II) above; calculating a time difference in time at which the expression levels are maximized between the model data and the three-point sampling data; and calculating three points of time at which an average of the time difference is less than 0.6 and a standard error of the time difference is less than 0.4, and collecting a biological specimen three times within 24 hours at those three points of time as collecting times. In particular, a biological rhythm can be predicted with high precision by collecting a biological specimen from a subject three times within 24 hours at eight-hour intervals.

In this method for predicting a biological rhythm, the clock genes can be a Per3 gene and an Nrld2 gene.

In general, the "phase" means a quantity that characterizes, in one period, the positions and states of points such as peaks and troughs in a periodic change. The circadian cycle of a change in the expression level of a clock gene can be substantially observed as a cosine wave function represented by formula (III) below. Herein, in the formula, E(t) is an expression level of the clock gene at a time t, A is an amplitude of the expression level, and C is an offset value.

$$E(t) = A\cos\left\{\frac{2\pi(t+\varpi)}{24}\right\} + C \qquad \cdots \text{ Formula (III)}$$

In the present invention, the "phase" is given by the inside of the cosine in the formula (III) and specifically indicates "$2\pi(t + \omega)/24$" in the formula (III). In addition, the "initial phase" is "$\omega$" that determines the phase at a time of t = 0. Furthermore, the "phase difference" is a difference in initial phase $\omega$ between clock genes.

According to the present invention, there is provided a method that achieves the prediction of a biological rhythm with high precision while minimizing the number of times a biological specimen is collected from a subject.

Brief Description of Drawings

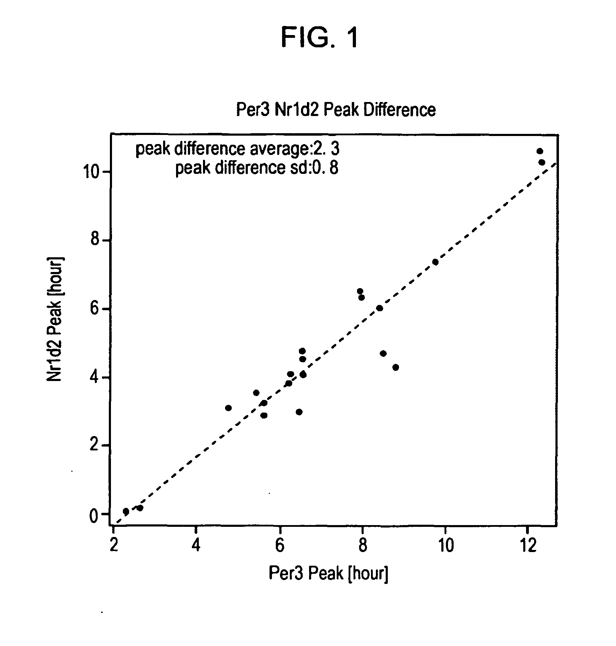

[Fig. 1] Fig. 1 is a diagram obtained by plotting the times at which expression levels are maximized in the circadian cycles of a change in expression levels of a Per3 gene and an Nrld2 gene (Example 1). The X-axis (horizontal axis) shows a time at which the expression level of the Per3 gene is maximized and the Y-axis (vertical axis) shows a time at which the expression level of the Nrld2 gene is maximized. In the drawing, the dotted line indicates a plot position in the case where the time difference (phase difference) between the times is 2 hours.

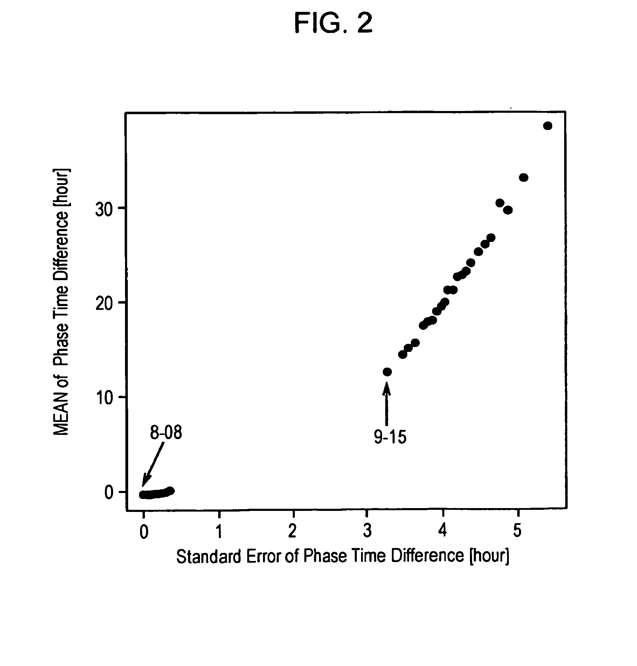

[Fig. 2] Fig. 2 is a diagram obtained by plotting the time differences in time at which the expression levels are maximized between model data and the circadian cycles calculated at time intervals at which sampling is performed three times (Example 2). In the drawing, the X-axis (horizontal axis) shows the standard error of the time differences at the sampling time intervals and the Y-axis (vertical axis) shows the average.

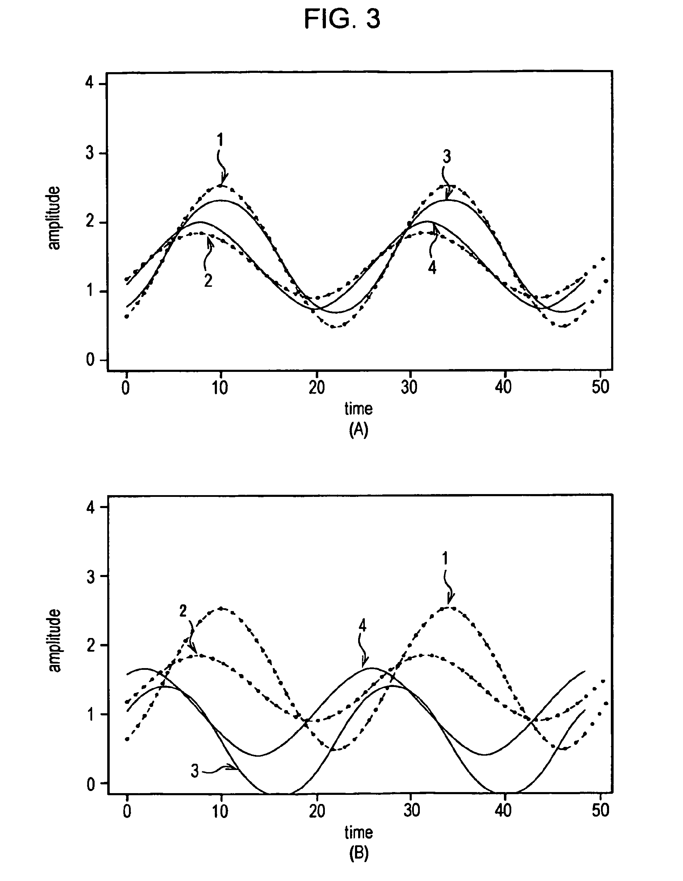

[Fig. 3] Fig. 3 is a diagram showing the circadian cycle of a change in the expression level of the Per3 calculated by performing all patterns of three-point sampling of the time interval "8:08" (A) or the time interval "9:15" (B) from the model data (Example 2). In the drawing, the symbol 1 denotes a circadian cycle of the Per3 of the model data and the symbol 2 denotes a circadian cycle of the Nrld2 of the model data. Furthermore, the symbol 3 denotes a circadian cycle of the Per3 of the three-point sampling data and the symbol 4 denotes a circadian cycle of the Nrld2 of the three-point sampling data.

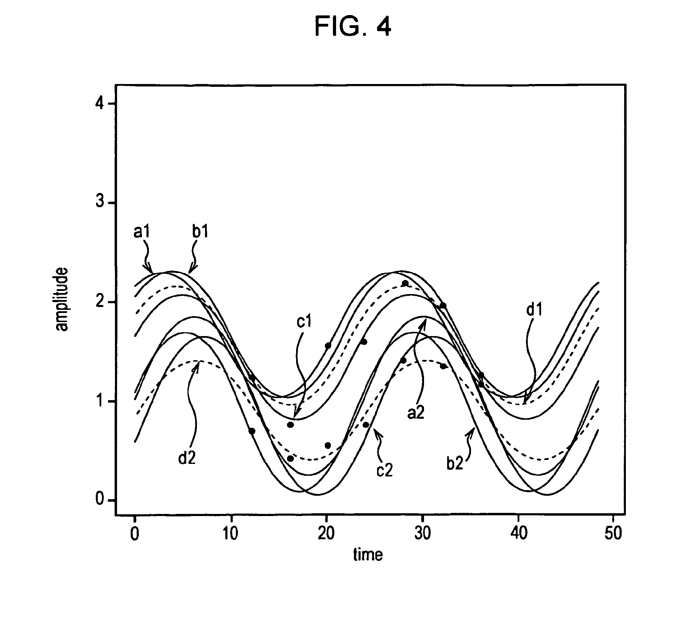

[Fig. 4] Fig. 4 is a diagram showing the circadian cycles of a change in the expression levels of the Per3 gene and the Nrld2 gene calculated by performing three-point sampling at eight-hour intervals (Example 3). In the drawing, the symbols a1, b1, and c1 respectively denote the circadian cycles of the Per3 gene in three-point sampling patterns a, b, and c, and the symbols a2, b2, and c2 respectively denote the circadian cycles of the Nrld2 gene in three-point sampling patterns a, b, and c. Furthermore, the symbols d1 and d2 denote the circadian cycles of the Per3 gene and the Nrld2 obtained through seven-point sampling.

Best Mode for Carrying Out the Invention

1. Method for predicting biological rhythm

The inventors of the present invention have considered that the number of times a biological specimen is collected from a subject can be reduced by predicting a biological rhythm using the difference in the cycle of a change in expression levels between a plurality of clock genes, and have examined the consideration. Then, the inventors have found that, by measuring two clock genes having different phases of circadian cycles of a change in expression levels, a biological rhythm can be predicted with high precision through only three times of collections of a biological specimen from the subject within 24 hours. That is, in the method for predicting a biological rhythm according to the present invention, the expression levels of two clock genes having different phases of circadian cycles of a change in expression levels are measured for biological specimens collected from a subject three times within 24 hours, and the biological rhythm of the subject is predicted on the basis of the obtained time series expression level data.

When two clock genes a and b have different phases of circadian cycles of a change in expression levels and the phase difference is assumed to be "$\theta$", the circadian cycles of the change in the expression levels of the clock genes can be modeled using cosine curve formulas (I) and (II) below.

$$E_a(t) = A_a \cos\left\{\frac{2\pi(t + \varpi)}{24}\right\} + C_a \qquad \cdots \text{Formula (I)}$$

$$E_b(t) = A_b \cos\left\{\frac{2\pi(t + \varpi + \theta)}{24}\right\} + C_b \qquad \cdots \text{Formula (II)}$$

Herein, in the formula (I), Ea(t), Aa, ω, and Ca are an expression level, an amplitude, an initial phase, and an offset value of the clock gene a at a time t. Furthermore, in the formula (II), Eb(t), Ab, and Cb are an expression level, an amplitude, and an offset value of the clock gene b at a time t.

In the case where the phase difference θ between the clock genes a and b is known, the cosine curve formulas (I) and (II) are formulas including five unknown constants (ω, Aa, Ab, Ca, and Cb). By measuring the expression levels Ea(t) and Eb(t) of the clock genes a and b in a biological specimen collected from a subject at a time t, two equations can be obtained from the cosine curve formulas (I) and (II). Therefore, with this model, when six equations are obtained from the cosine curve formulas (I) and (II) by collecting (sampling) biological specimens at three different times t, the circadian cycles of the change in the expression levels of the clock genes a and b can be calculated.

Mathematically, five equations are required to find five unknowns. Herein, by modeling the circadian cycles of a change in the expression levels of clock genes a and b having a known phase difference θ therebetween using the cosine curve formulas (I) and (II) above, five unknowns are calculated based on six equations. That is, by assuming the phase difference θ between the clock genes a and b to be a constraint, unknowns ω, Aa, Ab, Ca, and Cb can be calculated more accurately. Thus, the circadian cycle of a clock gene expression level, which is a biological phenomenon that is normally difficult to perform accurate evaluation, can be calculated with high precision.

More specifically, the method for predicting a biological rhythm includes (1) a step of collecting a biological specimen from a subject three times within 24 hours; (2) a step of measuring expression levels of two clock genes in the biological specimen, the two clock genes having different phases of circadian cycles of a change in expression levels; and (3) a step of calculating the circadian cycles of the change in the expression levels of the clock genes from time series expression level data obtained through these steps.

In the method for predicting a biological rhythm according to the present invention, examples of the target subject widely includes laboratory animals such as mice, rats, and monkeys in addition to humans.

2. Collection of biological specimen

The biological specimen collected from a subject is not particularly limited as long as the biological specimen is a biological tissue containing a gene expression product (mRNA) of a clock gene. In terms of ease of collection, the biological tissue can be preferably collected from a body surface such as a hair, an oral mucous membrane, or a skin.

A hair can be sampled by evulsion. The root of the evulsed hair includes hair follicle cells, and clock gene mRNA in the cells can be measured. Herein, the "hair follicle cells" are a group of cells that form inner root sheath, outer root sheath, and papilla that adhere to the root of evulsed body hairs.

For example, in the case where humans are targeted, the sampling site of hairs is not particularly limited, and head hairs, beards, hairs of arms or legs, or the like can be used. To suppress the variation in the measurement, the collection is preferably performed at the sites close to each other every time.

In the case of humans, the approximate number of hairs sampled each time is 5 to 10 for head hairs, 3 to 5 for beards, and 10 to 20 for hairs of arms. By using the number of hairs that is more than or equal to the above-described number of hairs, a sufficient amount of mRNA required to quantitatively determine the expression levels of the clock genes can be extracted.

The oral mucous membrane can be sampled by being scraped off from the surface of the oral mucous membrane using, for example, a brush or a spatula. Thus, clock gene mRNA in the scraped oral mucous membrane cell can be measured.

The sampling site of the oral mucous membrane is suitably a mucous membrane located at the inner lining of the cheek. To suppress the variation in the measurement, the oral mucous membrane is preferably collected from mucous membranes of both left and right sides.

The inventors of the present invention have examined the time interval between times of the sampling performed three times to achieve highly precise prediction of a biological rhythm. As a result, they have found that high precision can be achieved by collecting a biological specimen from a subject three times within 24 hours at eight-hour intervals (refer to Example 2).

In other words, in the method for predicting a biological rhythm according to the present invention, a biological rhythm can be predicted with the highest precision by performing sampling three times so that the time interval between the first and second sampling times and the time interval between the second and third sampling times are both eight hours.

3. Measurement of expression level

The expression level of a clock gene in a biological specimen can be measured by a publicly known method. For example, RNA is extracted from a biological specimen using a commercially available RNA extraction kit, and cDNA is synthesized through a reverse transcription reaction that uses the extracted RNA as a template. Then, an expression level is quantitatively determined using the cDNA by a comprehensive analytical method such as DNA microarray (DNA chip) or an individual analytical method such as real time PCR.

The clock genes to be measured may be a group of clock genes that have been identified so far. Representative examples of the clock genes include Per3 gene (NCBI Accession No. NM_016831), Per2 gene (NM_022817), Bmal1 gene (NM_001030272), Npas2 gene (NM_002518), Nrld2 gene (NM_021724), Nrld2 gene (NM_005126), Dbp gene (NM_001352), and Cryl gene (NM_004075).

In the method for predicting a biological rhythm according to the present invention, the expression levels of two genes among these clock genes are measured to obtain time series expression level data, the two genes having different phases of diurnal variations of a change in expression levels. Herein, in the case where organisms other than humans are targeted, the expression levels of homologues (homologous genes) of the above-described human clock genes in the organisms targeted are measured.

A combination of two clock genes can be freely selected as long as they have different phases of diurnal variations of a change in expression levels. The phases of diurnal variations of a change in the expression levels of two clock genes freely selected can be determined, for example, by the method described in Example 1 below.

The Per3 gene and the Nrld2 gene can be used as the preferable combination of two clock genes. The Per3 gene and the Nrld2 gene are stably expressed in a biological specimen and have large diurnal variations (amplitudes) in expression levels. Therefore, a biological rhythm can be accurately predicted by calculating the circadian cycles of a change in the expression levels of the Per3 gene and the Nrld2 gene from these time series expression level data.

4. Calculation of circadian cycle

The circadian cycles of a change in the expression levels of clock genes are calculated using formulas (I) and (II) below from the time series expression level data that shows the change in gene expression levels over time.

$$E_a(t) = A_a \cos\left\{\frac{2\pi(t+\varpi)}{24}\right\} + C_a \qquad \cdots \text{ Formula (I)}$$

$$E_b(t) = A_b \cos\left\{\frac{2\pi(t+\varpi+\theta)}{24}\right\} + C_b \qquad \cdots \text{ Formula (II)}$$

Herein, in the formula (I), Ea(t), Aa, ω, and Ca are an expression level, an amplitude, an initial phase, and an offset value of the clock gene a at a time t. Furthermore, in the formula (II), Eb(t), Ab, and Cb are an expression level, an amplitude, and an offset value of the clock gene b at a time t. Furthermore, θ is a phase difference between the two clock genes.

Six equations can be obtained using the formulas (I) and (II) above from the time series expression level data of the two clock genes obtained by performing sampling three times. From these six equations, five unknowns ω, Aa, Ab, Ca, and Cb are obtained by a conjugate gradient method or the like. Thus, the circadian cycles of a change in the expression levels of clock genes can be calculated, the circadian cycles reflecting the biological rhythm of a subject.

As described above, in the method for predicting a biological rhythm according to the present invention, the expression levels of two clock genes having different phases of circadian cycles of a change in expression levels are measured through three-point sampling, whereby the circadian cycles of a change in expression levels are calculated with high precision and thus the biological rhythm of a subject can be predicted.

By setting the number of times a biological specimen is collected from a subject to be only three within 24 hours, the burden on the subject imposed when the biological specimen is collected can be reduced. Moreover, by performing three-point sampling at eight-hour intervals, the biological specimen can be collected only in a time period when the subject is awake. Therefore, the biological rhythm can be accurately predicted without affecting the sleep/wake cycle of the subject.

[Example 1]

1. Determination of phase difference between Per3 gene and Nrld2 gene

In this Example, Per3 gene (hereinafter, simply referred to as "Per3") and Nrld2 gene (hereinafter, referred to as "Nrld2") were selected as two clock genes having different phases of circadian cycles of a change in expression levels, and the phase difference between the circadian cycles of a change in the expression levels of the genes was determined.

Head hairs were collected from 15 males and females who were 20 to 50 years old. The root of hairs including hair follicle cells attached thereto were quickly immersed in a cell lysis buffer (RNeasy Microkit: QIAGEN) to prepare a cell lysis solution. The hairs were collected at intervals of 3 to 4 hours, and 5 to 20 hairs were collected at each collecting time.

Total RNA was extracted from the cell lysis solution stored at -70°C in accordance with the protocol included with the cell lysis buffer and a reverse transcription reaction was performed. Real time PCR was performed using the reverse transcription product in an amount of one twentieth of the product to quantitatively determine the expression levels of the Per3 and the Nrld2. The real time PCR was performed with PRISM 7300 (ABI) using SYBR Green (ABI) or TaqMan MGB probe (ABI). The expression levels of the Per3 and the Nrld2 were corrected based on the expression level of 18S-rRNA, which was the internal standard, to obtain time series expression level data.

Cosine fitting of cosine curve formula (IV) below having a period of 24 hours was performed on the obtained time series expression level data by a nonlinear least squares method to obtain the phase difference between the circadian cycles of a change in the expression levels of the Per3 and the Nrld2.

$$E(t) = A\cos\left\{\frac{2\pi(t+\varpi)}{24}\right\} + C \qquad \cdots \text{ Formula (IV)}$$

(In the formula, E(t) is an expression level at a time t, A is an amplitude of the expression level, $\omega$ is an initial phase, and C is an offset value.)

Fig. 1 shows the results. The drawing is obtained by plotting the times t at which the expression level E(t) was maximized in the cosine curve formula (IV) after cosine fitting was conducted. The time t at which the expression level E(t) of the Per3 was maximized is plotted on the X-axis and the time t at which the expression level E(t) of the Nrld2 was maximized is plotted on the Y-axis.

As shown in Fig. 1, regarding the 15 subjects, the time t at which the expression level E(t) of the Per3 was maximized was widely spread from 0 to 12. Similarly, the time t at which the expression level E(t) of the Nrld2 was maximized was widely spread from 0 to 12.

However, the time difference (phase difference) between the time t at which the expression level E(t) of the Per3 was maximized and the time t at which the expression level E(t) of the Nrld2 was maximized was approximately 2 hours for each of the subjects. In Fig. 1, the dotted line indicates a plot position in the case where the phase difference between the Per3 and the Nrld2 is 2 hours.

The average and standard deviation of the phase difference between the Per3 and the Nrld2 obtained from the 15 subjects were 2.3 and 0.8, respectively. With this phase difference (hereinafter, referred to as "phase difference $\theta$"), the circadian cycles of a change in the expression levels of the Per3 and the Nrld2 were modeled using cosine curve formulas (V) and (VI) below, respectively.

$$E_{per3}(t) = A_{per3}\cos\left\{\frac{2\pi(t+\varpi)}{24}\right\} + C_{per3} \qquad \cdots \quad \text{Formula (V)}$$

(In the formula, Eper3(t) is a Per3 expression level at a time t, Aper3 is an amplitude of the expression level, ω is an initial phase of the Per3, and Cper3 is an offset value.)

$$E_{nrld2}(t) = A_{nrld2}\cos\left\{\frac{2\pi(t+\varpi+\theta.)}{24}\right\} + C_{nrld2} \qquad \cdots \quad \text{Formula (VI)}$$

(In the formula, Enrld2(t) is an Nrld2 expression level at a time t, Anrld2 is an amplitude of the expression level, θ is a phase difference between the circadian cycles of a change in the expression levels of the Per3 and Nrld2, and Cnrld2 is an offset value.)

[Example 2]

2. Examination of sampling time interval

When the cosine curve formulas (V) and (VI) above are used, the circadian cycle of a change in the expression level of the Per3 or the Nrld2 can be calculated by performing sampling at least three times and thus the biological rhythm of a subject can be estimated. Thus, in this Example, the time interval between times of the sampling performed three times was determined so that a biological rhythm can be estimated with the highest precision. Assuming that the sampling was performed three times within 24 hours, all the conceivable sampling time intervals (276 patterns) were examined.

First, the hourly changes in the expression levels of the two genes were calculated from the cosine curve formula (IV) obtained in Example 1 by performing cosine fitting on the time series expression level data of the Per3 and the Nrld2. Then, three arbitrary points of time t and expression levels V(t) at those points of time were extracted as three-point sampling data from the calculated model data that shows the hourly changes in the expression levels.

These three arbitrary points of time t and the expression levels v(t) at those points of time were substituted into the formulas (V) and (VI), and the amplitudes Aper3 and Anrld2 of the expression levels, the initial phase ω of the Per3, and the offset values Cper3 and Cnrld2 were obtained by a conjugate gradient method.

The conjugate gradient method was performed through the process below. That is, first, the sum of squares d of the expression levels v(t) substituted into the formulas (V) and (VI) and the expression levels E(t) of the model data that shows the hourly changes in the expression levels was defined as a distance between the three-point sampling data and the model data using formula (VII) below.

$$d = \sum\{v(t)-E(t)\}^2 \qquad \cdots \quad \text{Formula (VII)}$$

Then, the amplitudes Aper3 and Anrld2, the initial phase ω, and the offset values Cper3 and Cnrld that minimize the distance d were obtained by the conjugate gradient method. Note that, in the case where a nonlinear least squares method was employed, these unknown constants could not be obtained because the distance d did not converge to a minimum value.

Herein, when the conjugate gradient method is employed, the initial values of the unknown constants significantly affect constants to be obtained. Thus, if appropriate initial values are not set, the distance d converges to a local extreme and thus proper circadian cycles cannot be obtained.

Therefore, in this case, the average of the amplitude A of the model data that shows the hourly changes in the expression levels was set as appropriate initial values of the amplitudes Aper3 and Anrld2. Moreover, the average of the three-point sampling data was set as initial values of the offset values Cper3 and Cnrld2. Note that, regarding the amplitude A of the model data, the amplitude Aper3 of the Per3 was 0.8014513 and the amplitude Anrld2 of the Nrld2 was 0.6402411.

Furthermore, the initial phase ω of the Per3 was limited to an integer of 0 to 23 to ease the operational analysis.

Then, the initial phase ω that can provide the minimum distance d was obtained by giving an integer of 0 to 23 as an initial value one by one and applying the conjugate gradient method.

As described above, there was obtained the time difference between the time t at which the expression level v(t) was maximized in the formula (V) in which the amplitude Aper3 of the expression level, the initial phase w of the Per3, and the offset value Cper3 were determined and the time at which the expression level v(t) was maximized in the model data. Then, the time interval between the sampling times was examined so that the time difference was minimized.

All the hourly times from 0 to 23 were set as the first sampling times. Then, the combinations (276 patterns) of the time intervals between the three sampling times were examined so that the second sampling and the third sampling were completed within 24 hours from the first sampling. Regarding the combinations of all the time intervals, the average and standard error of the time difference were obtained. The standard error indicates how much the circadian cycle calculated at the sampling time interval depends on the first sampling time. Furthermore, the average indicates the precision of the calculated circadian cycle. Thus, it can be said that the calculated circadian cycle becomes more accurate as the standard error and average are decreased.

"Table 1" to "Table 5" show the average and standard error obtained regarding the combinations of all the time intervals. The combinations of the time intervals are listed in "Table 1" to "Table 5" in the order of time intervals having a lower standard error and average. The time interval is shown by combining the time interval between the first and second sampling times with the time interval between the second and third sampling times. For example, "13:06" in the drawing indicates that the time interval between the first and second sampling times is 13 hours and the time interval between the second and third sampling times is 6 hours.

[Table 1]

| Time interval | Standard error | Average | Time interval | Standard error | Average | Time interval | Standard error | Average |
|---|---|---|---|---|---|---|---|---|
| 8:08 | 0.000 | 0.127 | 5:10 | 0.045 | 0.130 | 13:05 | 0.057 | 0.134 |
| 7:09 | 0.017 | 0.129 | 9:10 | 0.045 | 0.130 | 5:06 | 0.057 | 0.134 |
| 9:08 | 0.017 | 0.129 | 10:05 | 0.045 | 0.128 | 4:10 | 0.062 | 0.131 |
| 8:07 | 0.017 | 0.129 | 5:09 | 0.045 | 0.128 | 10:10 | 0.062 | 0.132 |
| 9:07 | 0.017 | 0.127 | 10:09 | 0.045 | 0.130 | 10:04 | 0.062 | 0.131 |
| 7:08 | 0.017 | 0.127 | 9:05 | 0.045 | 0.129 | 9:04 | 0.062 | 0.131 |
| 8:09 | 0.017 | 0.127 | 6:12 | 0.046 | 0.129 | 4:11 | 0.062 | 0.131 |
| 7:07 | 0.026 | 0.129 | 6:06 | 0.046 | 0.129 | 11:09 | 0.062 | 0.132 |
| 7:10 | 0.026 | 0.129 | 12:06 | 0.046 | 0.129 | 4:09 | 0.063 | 0.132 |
| 10:07 | 0.026 | 0.129 | 5:11 | 0.047 | 0.129 | 9:11 | 0.063 | 0.131 |
| 9:06 | 0.030 | 0.128 | 11:08 | 0.047 | 0.128 | 11:04 | 0.063 | 0.133 |
| 6:09 | 0.030 | 0.129 | 8:05 | 0.047 | 0.128 | 12:08 | 0.064 | 0.134 |
| 9:09 | 0.030 | 0.127 | 11:05 | 0.048 | 0.128 | 4:12 | 0.064 | 0.132 |
| 6:08 | 0.032 | 0.127 | 8:11 | 0.048 | 0.129 | 8:04 | 0.065 | 0.133 |
| 10:06 | 0.032 | 0.127 | 5:08 | 0.048 | 0.130 | 8:12 | 0.065 | 0.133 |
| 8:10 | 0.032 | 0.127 | 7:12 | 0.051 | 0.131 | 4:08 | 0.065 | 0.132 |
| 8:06 | 0.032 | 0.127 | 12:05 | 0.051 | 0.130 | 12:04 | 0.065 | 0.132 |
| 6:10 | 0.032 | 0.127 | 5:07 | 0.051 | 0.131 | 5:05 | 0.066 | 0.132 |
| 10:08 | 0.032 | 0.128 | 5:12 | 0.051 | 0.131 | 14:05 | 0.066 | 0.133 |
| 11:07 | 0.037 | 0.129 | 12:07 | 0.051 | 0.130 | 5:14 | 0.066 | 0.133 |
| 6:11 | 0.037 | 0.129 | 7:05 | 0.051 | 0.130 | 13:04 | 0.067 | 0.134 |
| 7:06 | 0.038 | 0.129 | 5:13 | 0.057 | 0.131 | 7:13 | 0.067 | 0.133 |
| 11:06 | 0.038 | 0.129 | 13:06 | 0.057 | 0.130 | 4:07 | 0.067 | 0.132 |

(continued)

| Time interval | Standard error | Average | Time interval | Standard error | Average | Time interval | Standard error | Average |
|---|---|---|---|---|---|---|---|---|
| 7:11 | 0.038 | 0.129 | 6:05 | 0.057 | 0.131 | 4:13 | 0.068 | 0.133 |
| 6:07 | 0.038 | 0.129 | 6:13 | 0.057 | 0.134 | 7:04 | 0.068 | 0.132 |

[Table 2]

| Time interval | Standard error | Average | Time interval | Standard error | Average | Time interval | Standard error | Average |
|---|---|---|---|---|---|---|---|---|
| 13:07 | 0.068 | 0.133 | 13:03 | 0.084 | 0.136 | 16:03 | 0.094 | 0.141 |
| 6:14 | 0.071 | 0.133 | 8:13 | 0.084 | 0.135 | 5:16 | 0.094 | 0.142 |
| 14:04 | 0.071 | 0.133 | 3:08 | 0.084 | 0.135 | 3:16 | 0.097 | 0.142 |
| 4:06 | 0.071 | 0.133 | 13:08 | 0.084 | 0.135 | 16:05 | 0.098 | 0.139 |
| 6:04 | 0.072 | 0.133 | 3:13 | 0.085 | 0.137 | 5:03 | 0.098 | 0.139 |
| 4:14 | 0.072 | 0.133 | 8:03 | 0.085 | 0.137 | 4:17 | 0.100 | 0.139 |
| 14:06 | 0.072 | 0.133 | 7:14 | 0.085 | 0.137 | 3:04 | 0.100 | 0.143 |
| 4:05 | 0.077 | 0.134 | 14:03 | 0.085 | 0.139 | 17:03 | 0.100 | 0.143 |
| 5:15 | 0.077 | 0.134 | 3:07 | 0.085 | 0.139 | 3:03 | 0.105 | 0.146 |
| 15:04 | 0.077 | 0.134 | 3:14 | 0.086 | 0.139 | 18:03 | 0.105 | 0.146 |
| 5:04 | 0.077 | 0.134 | 14:07 | 0.086 | 0.136 | 3:18 | 0.105 | 0.145 |
| 15:05 | 0.077 | 0.136 | 7:03 | 0.086 | 0.136 | 17:05 | 0.106 | 0.142 |
| 4:15 | 0.077 | 0.135 | 4:16 | 0.087 | 0.136 | 16:06 | 0.106 | 0.143 |
| 10:11 | 0.082 | 0.135 | 4:04 | 0.087 | 0.137 | 6:02 | 0.106 | 0.143 |
| 3:10 | 0.082 | 0.135 | 16:04 | 0.087 | 0.137 | 2:16 | 0.107 | 0.145 |
| 11:03 | 0.082 | 0.135 | 3:15 | 0.088 | 0.137 | 5:02 | 0.107 | 0.144 |
| 3:11 | 0.083 | 0.137 | 15:06 | 0.088 | 0.137 | 2:17 | 0.107 | 0.145 |
| 11:10 | 0.083 | 0.135 | 6:03 | 0.088 | 0.137 | 4:02 | 0.108 | 0.140 |
| 10:03 | 0.083 | 0.136 | 6:15 | 0.089 | 0.138 | 2:18 | 0.108 | 0.140 |
| 9:12 | 0.083 | 0.135 | 15:03 | 0.089 | 0.136 | 2:01 | 0.108 | 0.141 |
| 3:09 | 0.083 | 0.137 | 3:06 | 0.089 | 0.140 | 18:04 | 0.109 | 0.142 |
| 12:03 | 0.083 | 0.137 | 17:04 | 0.091 | 0.139 | 1:20 | 0.109 | 0.143 |
| 12:09 | 0.084 | 0.138 | 4:03 | 0.092 | 0.136 | 3:01 | 0.109 | 0.143 |
| 3:12 | 0.084 | 0.136 | 3:17 | 0.092 | 0.136 | 20:03 | 0.110 | 0.143 |
| 9:03 | 0.084 | 0.136 | 3:05 | 0.094 | 0.141 | 2:03 | 0.110 | 0.143 |

[Table 3]

| Time interval | Standard error | Average | Time interval | Standard error | Average | Time interval | Standard error | Average |
|---|---|---|---|---|---|---|---|---|
| 3:19 | 0.110 | 0.143 | 20:02 | 0.136 | 0.165 | 1:14 | 0.160 | 0.167 |
| 19:02 | 0.111 | 0.142 | 8:02 | 0.136 | 0.171 | 15:02 | 0.160 | 0.170 |

(continued)

| Time interval | Standard error | Average | Time interval | Standard error | Average | Time interval | Standard error | Average |
|---|---|---|---|---|---|---|---|---|
| 2:04 | 0.111 | 0.143 | 2:05 | 0.136 | 0.170 | 7:15 | 0.162 | 0.170 |
| 18:02 | 0.111 | 0.143 | 10:12 | 0.138 | 0.171 | 2:07 | 0.162 | 0.169 |
| 4:18 | 0.112 | 0.143 | 2:10 | 0.139 | 0.167 | 21:02 | 0.162 | 0.166 |
| 18:05 | 0.113 | 0.141 | 12:02 | 0.139 | 0.168 | 22:01 | 0.165 | 0.166 |
| 1:18 | 0.113 | 0.141 | 7:02 | 0.140 | 0.162 | 15:08 | 0.165 | 0.162 |
| 5:01 | 0.114 | 0.141 | 2:15 | 0.141 | 0.166 | 1:15 | 0.165 | 0.163 |
| 2:19 | 0.114 | 0.142 | 15:07 | 0.142 | 0.168 | 8:01 | 0.170 | 0.176 |
| 3:02 | 0.114 | 0.142 | 7:01 | 0.144 | 0.170 | 6:17 | 0.171 | 0.179 |
| 19:03 | 0.116 | 0.144 | 16:07 | 0.144 | 0.165 | 1:06 | 0.171 | 0.181 |
| 2:11 | 0.116 | 0.146 | 1:16 | 0.145 | 0.167 | 17:01 | 0.172 | 0.175 |
| 11:02 | 0.116 | 0.146 | 2:12 | 0.147 | 0.168 | 21:01 | 0.173 | 0.182 |
| 11:11 | 0.118 | 0.145 | 12:10 | 0.148 | 0.168 | 8:15 | 0.173 | 0.179 |
| 1:21 | 0.118 | 0.144 | 10:02 | 0.148 | 0.166 | 15:01 | 0.176 | 0.175 |
| 16:02 | 0.119 | 0.147 | 2:14 | 0.151 | 0.174 | 1:08 | 0.177 | 0.169 |
| 2:06 | 0.122 | 0.148 | 14:08 | 0.151 | 0.175 | 6:01 | 0.177 | 0.170 |
| 6:16 | 0.122 | 0.148 | 13:09 | 0.151 | 0.176 | 17:06 | 0.179 | 0.175 |
| 17:02 | 0.122 | 0.145 | 2:13 | 0.153 | 0.167 | 1:17 | 0.180 | 0.179 |
| 5:17 | 0.126 | 0.146 | 9:13 | 0.154 | 0.165 | 1:22 | 0.180 | 0.181 |
| 2:20 | 0.126 | 0.146 | 2:09 | 0.154 | 0.166 | 1:01 | 0.183 | 0.176 |
| 14:02 | 0.127 | 0.147 | 13:02 | 0.157 | 0.175 | 20:01 | 0.183 | 0.172 |
| 2:08 | 0.130 | 0.151 | 9:02 | 0.157 | 0.176 | 3:20 | 0.184 | 0.172 |
| 8:14 | 0.130 | 0.152 | 9:01 | 0.157 | 0.171 | 1:03 | 0.187 | 0.172 |
| 2:02 | 0.130 | 0.152 | 14:09 | 0.160 | 0.166 | 18:01 | 0.188 | 0.179 |

[Table 4]

| Time interval | Standard error | Average | Time interval | Standard error | Average |
|---|---|---|---|---|---|
| 1:05 | 0.188 | 0.178 | 1:02 | 0.209 | 0.187 |
| 5:18 | 0.191 | 0.176 | 1:19 | 0.210 | 0.189 |
| 19:01 | 0.191 | 0.173 | 19:04 | 0.213 | 0.184 |
| 4:19 | 0.192 | 0.171 | 4:01 | 0.213 | 0.185 |
| 1:13 | 0.195 | 0.188 | 1:04 | 0.215 | 0.179 |
| 10:01 | 0.195 | 0.186 | 13:01 | 0.288 | 0.328 |
| 2:21 | 0.196 | 0.194 | 1:10 | 0.289 | 0.327 |
| 13:10 | 0.199 | 0.176 | 10:13 | 0.290 | 0.331 |
| 1:09 | 0.200 | 0.175 | 1:12 | 0.321 | 0.445 |
| 14:01 | 0.200 | 0.172 | 11:01 | 0.322 | 0.444 |
| 9:14 | 0.203 | 0.179 | 12:11 | 0.323 | 0.443 |

(continued)

| Time interval | Standard error | Average | Time interval | Standard error | Average |
|---|---|---|---|---|---|
| 16:01 | 0.203 | 0.180 | 1:11 | 0.355 | 0.515 |
| 1:07 | 0.204 | 0.180 | 12:01 | 0.355 | 0.516 |
| 7:16 | 0.208 | 0.181 | 11:12 | 0.356 | 0.514 |

[Table 5]

| Time interval | Standard error | Average |
|---|---|---|
| 9:15 | 3.27 | 12.78 |
| 8:16 | 3.47 | 14.57 |
| 23:01 | 3.55 | 15.25 |
| 16:08 | 3.64 | 15.82 |
| 2:22 | 3.75 | 17.57 |
| 22:02 | 3.82 | 17.96 |
| 1:23 | 3.87 | 18.17 |
| 6:18 | 3.92 | 18.96 |
| 14:10 | 3.98 | 19.57 |
| 20:04 | 4.03 | 20.02 |
| 12:12 | 4.08 | 21.25 |
| 11:13 | 4.15 | 21.23 |
| 3:21 | 4.20 | 22.66 |
| 21:03 | 4.26 | 22.89 |
| 19:05 | 4.31 | 23.21 |
| 13:11 | 4.38 | 24.09 |
| 15:09 | 4.48 | 25.25 |
| 7:17 | 4.57 | 26.00 |
| 17:07 | 4.65 | 26.59 |
| 5:19 | 4.77 | 30.21 |
| 4:20 | 4.87 | 29.47 |
| 10:14 | 5.08 | 32.81 |
| 18:06 | 5.40 | 38.23 |

Moreover, Fig. 2 is a diagram in which each of the combinations of the time intervals is plotted by taking the standard error on the X-axis and the average on the Y-axis.

As shown in "Table 1" to "Table 5" and Fig. 2, the standard error and average were minimized in the time interval "8:08". As is clear from this, the circadian cycle can be calculated with the highest precision by performing sampling three times so that the time interval between the first and second sampling times and the time interval between the second and third sampling times are both eight hours.

In addition, the standard error and average are sufficiently low in the combinations of the time intervals shown in "Table 1" to "Table 4". It is considered that, by employing these sampling time intervals, accurate circadian cycles can be calculated. On the other hand, it is found that the combinations of the time intervals shown in "Table 5" are unsuitable because the standard error and average are high.

Fig. 3 shows the circadian cycle of a change in the expression level of the Per3 calculated by performing all patterns of the three-point sampling of the time interval "8:08" (A) or the time interval "9:15" (B) from the model data. In the drawing, the symbol 1 denotes a circadian cycle of the Per3 of the model data and the symbol 2 denotes a circadian cycle of the Nrld2 of the model data. Furthermore, the symbol 3 denotes a circadian cycle of the Per3 of the three-point sampling data and the symbol 4 denotes a circadian cycle of the Nrld2 of the three-point sampling data.

As shown in Fig. 3(A), in the case where the time interval between the first and second sampling times and the time interval between the second and third sampling times are both eight hours, the circadian cycles of the Per3 and the Nrld2 between the model data and the three-point sampling data agree with each other well, which means the circadian cycles can be calculated with high precision.

On the other hand, as is clear from Fig. 3(B), in the case where the time interval between the first and second sampling times is nine hours and the time interval between the second and third sampling times is fifteen hours, the circadian cycles of the Per3 and the Nrld2 between the model data and the three-point sampling data do not agree with each other, which means the circadian cycles cannot be calculated.

[Example 3]

3. Estimation of biological rhythm through three-point sampling

It was found from the results in Example 2 that the circadian cycle could be calculated with the highest precision by performing sampling three times at eight-hour intervals. Thus, a biological rhythm was attempted to be predicted by actually performing three-point sampling at eight-hour intervals. The three-point sampling was performed using three patterns shown in "Table 6" below.

[Table 6]

|  | Sampling time | | |
| --- | --- | --- | --- |
|  | First | Second | Third |
| Three-point sampling a | 12:00 | 20:00 | 28:00 |
| Three-point sampling b | 16:00 | 24:00 | 32:00 |
| Three-point sampling c | 20:00 | 28:00 | 36:00 |

In three-point samplings a to c, the expression levels of the Per3 and the Nrld2 at each of the times were quantitatively determined by the method described in Example 1. Then, by the same method as described in Example 2, the three points of time t and the expression levels E(t) at those points of time were substituted into the formulas (V) and (VI), and the amplitudes Aper3 and Anrld2 of the expression levels, the initial phase $\omega$ of the Per3, and the offset values Cper3 and Cnrld2 were obtained by a conjugate gradient method. Thus, the circadian cycles of a change in the expression levels of the Per3 and Nrld2 were calculated.

Fig. 4 shows the results. In the drawing, the symbols a1, b1, and c1 respectively denote the circadian cycles of the Per3 gene in the three-point sampling patterns a, b, and c, and the symbols a2, b2, and c2 respectively denote the circadian cycles of the Nr1d2 gene in the three-point sampling patterns a, b, and c. Furthermore, the symbols d1 and d2 denote circadian cycles obtained by performing cosine fitting of the cosine curve formula (IV) above using a nonlinear least squares method on the time series expression level data obtained through seven samplings from 12:00 to 36:00. The symbol d1 denotes a circadian cycle of the Per3 and the symbol d2 denotes a circadian cycle of the Nrld2.

As shown in Fig. 4, in the three-point samplings a to c, the circadian cycles of a change in the expression levels of the Per3 and Nrld2 were calculated with high reproducibility. The error of the phase between the circadian cycles of a change in the expression levels of the Per3 and the Nrld2 through the three-point samplings a to c and the circadian cycles (refer to the symbols d1 and d2 in the drawing) through seven samplings was 0.75 hours on average.

It was found from this result that, by performing three-point sampling concerning the expression levels of the Per3 and Nrld2 having different phases of the circadian cycles of a change in expression levels, the circadian cycles of a change in expression levels were calculated, whereby the biological rhythm of a subject could be predicted with high precision.

Industrial Applicability

The method for predicting a biological rhythm according to the present invention can be used to achieve time medical care, exhibit one's ability, and lose weight. Furthermore, the method can be used to prevent various diseases caused by the shift of a biological rhythm and improve poor physical condition such as jet lag.

**Claims**

1. A method for predicting a biological rhythm of a subject on the basis of time series expression level data obtained by measuring expression levels of two clock genes in biological specimens collected from the subject three times within 24 hours, the clock genes having different phases of circadian cycles of a change in expression levels. comprising:

   (1) a step of collecting a biological specimen from a subject three times within 24 hours at eight-hour intervals;
   (2) a step of measuring expression levels of two clock genes in the biological specimen, the two clock genes having different phases of circadian cycles of a change in expression levels; and
   (3) a step of calculating the circadian cycles from time series expression level data obtained through the steps (1) and (2).

   wherein in step (1) the biological specimens are taken every time from a subject's biological tissue containing a gene expression product of said two clock genes,
   wherein in step (3) the circadian cycles are calculated from the time series expression level data using the following formulas (I) and (II) below,

$$E_a(t) = A_a \cdot \cos\left(\frac{2\pi \cdot (t + \omega)}{24}\right) + C_a \ . \qquad \text{Formula (I)}$$

$$E_b(t) = A_b \cdot \cos\left(\frac{2\pi \cdot (t + \omega + \theta)}{24}\right) + C_b \ . \qquad \text{Formula (II)}$$

   wherein in formula (I) the terms $E_a(t)$, $A_a$, $\omega$, and $C_a$ denote an expression level, an amplitude, an initial phase, and an offset value, respectively, of one of the two clock genes at a time t;
   wherein in formula (II), $E_b(t)$, $A_b$, and $C_b$ denote an expression level, an amplitude, and an offset value, respectively, of the other one of the two clock genes at a time t; and
   wherein $\theta$ denotes a phase difference between the two clock genes.

2. The method according to claim 1,
   comprising:

   calculating model data from a cosine curve obtained by performing cosine fitting on time series expression level data, the model data showing a change in expression level every hour;
   calculating three-point sampling data from three arbitrary points of time and expression levels at those points of time extracted from the model data, and the formulas (I) and (II) above;
   calculating a time difference in time at which the expression levels are maximized between the model data and the three-point sampling data; and
   calculating three points of time at which an average of the time difference is less than 0.6 and a standard error of the time difference is less than 0.4, and collecting a biological specimen three times within 24 hours at those three points of time as collecting times.

3. The method according to claim 1,
   wherein a Per3 gene and an Nrld2 gene are used as the clock genes.

**Patentansprüche**

1. Verfahren zur Vorhersage eines biologischen Rhythmus eines Individuums auf der Grundlage von durch Messen der Expressionsniveaus von zwei Uhrengenen in von dem Individuum dreimal innerhalb von 24 Stunden gesammelten biologischen Proben erhaltenen Expressionsniveau-Zeitreihendaten, wobei die Uhrengene unterschiedliche Phasen circadianer Zyklen einer Änderung der Expressionsniveaus aufweisen,
   umfassend:

   (1) einen Schritt des dreimaligen Sammelns einer biologischen Probe von einem Individuum innerhalb von 24

EP 2 336 355 B1

Stunden in achtstündigen Zeitabständen;

(2) einen Schritt des Messens der Expressionsniveaus von zwei Uhrengenen in der biologischen Probe, wobei die beiden Uhrengene unterschiedliche Phasen circadianer Zyklen einer Änderung der Expressionsniveaus aufweisen; und

(3) einen Schritt des Berechnens der circadianen Zyklen anhand über die Schritte (1) und (2) erhaltener Expressionsniveau-Zeitreihendaten, wobei in Schritt (1) die biologischen Proben jedesmal einem ein Genexpressionsprodukt der beiden Uhrengene enthaltenden biologischen Gewebe des Individuums entnommen werden,

wobei in Schritt (3) die circadianen Zyklen anhand der Expressionsniveau-Zeitreihendaten unter Verwendung der folgenden Formeln (I) und (II) unten berechnet werden,

$$E_a(t) = A_a \cdot \cos\left(\frac{2\pi \cdot (t + \omega)}{24}\right) + C_a \, . \qquad \text{Formel I}$$

$$E_b(t) = A_b \cdot \cos\left(\frac{2\pi \cdot (t + \omega + \theta)}{24}\right) + C_b \, . \qquad \text{Formel II}$$

wobei in Formel (I) die Ausdrücke $E_a(t)$, $A_a$, $\omega$ bzw. $C_a$ ein Expressionsniveau, eine Amplitude, eine Anfangsphase bzw. einen Offset-Wert eines der beiden Uhrengene zum Zeitpunkt t bezeichnen; wobei in Formel (II) $E_b(t)$, $A_b$ bzw. $C_b$ ein Expressionsniveau, eine Amplitude bzw. einen Offset-Wert des anderen der beiden Uhrengene zum Zeitpunkt t bezeichnen; und

wobei $\theta$ einen Phasenunterschied zwischen den beiden Uhrengenen bezeichnet.

2. Verfahren nach Anspruch 1,
umfassend:

Berechnen von Modelldaten anhand einer Cosinus-Kurve mittels Cosinus-Kurvenanpassung an Expressionsniveau-Zeitreihendaten, wobei die Modelldaten eine stündliche Änderung des Expressionsniveaus zeigen;
Berechnen von Drei-Punkte-Probenahme-Daten anhand aus den Modelldaten extrahierter drei willkürlicher Zeitpunkte und der Expressionsniveaus zu diesen drei Zeitpunkten sowie der obigen Formeln (I) und (II);
Berechnen eines Zeitunterschieds bei dem Zeitpunkt, an dem die Expressionsniveaus zwischen den Modelldaten und den Drei-Punkte-Probenahme-Daten maximiert sind; und
Berechnen von drei Zeitpunkten, an denen ein mittlerer Zeitunterschied weniger als 0,6 und ein Standardfehler des Zeitunterschieds weniger als 0,4 beträgt, und dreimaliges Sammeln einer biologischen Probe innerhalb von 24 Stunden zu diesen drei Zeitpunkten als Sammelzeiten.

3. Verfahren nach Anspruch 1,
wobei ein Per3-Gen und ein Nr1d2-Gen als Uhrengene eingesetzt werden.

**Revendications**

1. Procédé de prédiction d'un rythme biologique d'un sujet sur la base de données de niveau d'expression en série temporelle obtenues par mesure de niveaux d'expression de deux gènes d'horloge dans des échantillons biologiques collectés à partir du sujet trois fois en 24 heures, les gènes d'horloge ont différentes phases de cycles circadiens d'un changement des taux d'expression,
comprenant :

(1) une étape de collecte d'un spécimen biologique à partir d'un sujet trois fois en 24 heures à des intervalles de huit heures :
(2) une étape de mesure de niveaux d'expression de deux gènes d'horloge dans le spécimen biologique, les deux gènes d'horloge ayant différentes phases de cycles circadiens d'un changement de taux d'expression ; et
(3) une étape de calcul des cycles circadiens à partir des données de niveau d'expression en série temporelle obtenues à partir des étapes (1) et (2),

où, dans l'étape (1), les spécimens biologiques sont prélevés chaque fois à partir d'un tissu biologique d'un sujet contenant un produit d'expression génique desdits deux gènes d'horloge,

où, dans l'étape (3), les cycles circadiens sont calculés à partir des données de niveau d'expression en série temporelle en utilisant les formules (I) et (II) ci-dessous,

$$E_a(t) = A_a \cdot \cos\left(\frac{2\pi \cdot (t + \omega)}{24}\right) + C_a \qquad , \qquad \text{Formule (I)}$$

$$E_b(t) = A_b \cdot \cos\left(\frac{2\pi \cdot (t + \omega + \theta)}{24}\right) + C_b \qquad , \qquad \text{Formule (II)}$$

où, dans la formule (I), les termes $E_a(t)$, $A_a$, $\omega$, et $C_a$ désignent un niveau d'expression, une amplitude, une phase initiale, et une valeur de décalage, respectivement, d'un des deux gènes d'horloge à un temps t ;

où, dans la formule (II), $E_b(t)$, $A_b$, et $C_b$ désignent un niveau d'expression, une amplitude, et une valeur de décalage, respectivement, de l'autre des deux gènes d'horloge à un temps t ; et

dans lequel $\theta$ désigne un déphasage entre les deux gènes d'horloge.

2. Procédé selon la revendication 1,
comprenant :

le calcul de données de modèle à partir d'une courbe cosinusoïdale obtenue en effectuant un ajustement cosinusoïdal sur des données de niveau d'expression en série temporelle, les données de modèle indiquant un changement de niveau d'expression chaque heure ;
le calcul de données d'échantillonnage à trois points de temps arbitraires et de niveaux d'expression à ces temps extraits à partir des données de modèle, et les formules (I) et (II) ci-dessus ;
le calcul d'une différence de temps à laquelle les niveaux d'expression sont maximisés entre les données de modèle et les données d'échantillonnage à trois points ; et
le calcul de trois temps auxquels une moyenne de la différence de temps est inférieure à 0,6 et une erreur standard de la différence de temps est inférieure à 0,4, et la collecte d'un spécimen biologique trois fois en 24 heures à ces trois temps en tant que temps de collecte.

3. Procédé selon la revendication 1,
dans lequel un gène Per3 et un gène Nrld2 sont utilisés en tant que gènes d'horloge.

# FIG. 1

Per3 Nr1d2 Peak Difference

# FIG. 2

# FIG. 3

(A)

(B)

# FIG. 4

**EP 2 336 355 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009014036 A
- WO 2004012128 A